# EUROPÄISCHE PATENTSCHRIFT

(11) **EP 1 628 688 B1**
(45) Veröffentlichungstag und Bekanntmachung des Hinweises auf die Patenterteilung: **23.05.2007**
(21) Anmeldenummer: 04735879.1
(22) Anmeldetag: 03.06.2004
(51) Int. Cl.: A61L 2/14, H01T 19/04

(54) **BEHANDLUNG VON LEBENDE ZELLEN ENTHALTENDEN BIOLOGISCHEN MATERIALIEN MIT EINEM DURCH EINE GASENTLADUNG ERZEUGTEN PLASMA**
TREATMENT OF BIOLOGICAL MATERIAL CONTAINING LIVING CELLS USING A PLASMA GENERATED BY A GAS DISCHARGE
TRAITEMENT DE MATIERES BIOLOGIQUES CONTENANT DES CELLULES VIVANTES A L'AIDE D'UN PLASMA PRODUIT PAR UNE DECHARGE GAZEUSE

(30) Priorität: 03.06.2003 DE 10324926
(43) Veröffentlichungstag der Anmeldung: 01.03.2006
(73) Patentinhaber: Fachhochschule Hildesheim / Holzminden / Göttingen, 31134 Hildesheim (DE)
(72) Erfinder: VIÖL, Wolfgang, 37139 Adelebsen (DE)
(74) Vertreter: Rehberg Hüppe + Partner
(86) Internationale Anmeldenummer: PCT/EP2004/005988
(87) Internationale Veröffentlichungsnummer: WO 2004/105810

(56) Entgegenhaltungen:
- EP-A- 0 956 827
- WO-A-87/07248
- WO-A-20/04023927
- US-A- 5 866 082

## Beschreibung

Die Erfindung betrifft ein Verfahren zur Behandlung eines lebende Zellen enthaltenden biologischen Materials mit einem durch eine Gasentladung bei Atmosphärendruck erzeugten Plasma nach dem Oberbegriff des Patentanspruchs 1 sowie eine entsprechende Vorrichtung nach dem Oberbegriff des Patentanspruchs 11. Dabei ist ein Plasma im Sinne der vorliegenden Erfindung kein Blutplasma sondern ein physikalisches Plasma, d.h. ein bestimmter, elektrisch leitfähiger Zustand eines Gases bzw. eines Gasgemisches.

### STAND DER TECHNIK

Die Behandlung organischer Materialien mit einem Plasma, welches durch eine Gasentladung bei Atmosphärendruck erzeugt wird, ist beispielsweise durch der DE 199 57 775 C1 bekannt. Hier werden Holzoberflächen durch eine dielektrisch behinderte Entladung modifiziert. Hinweise zur Behandlung von biologischem Material, das lebende Zellen enthält, sind hier jedoch nicht gegeben.

Ein Verfahren und eine Vorrichtung, zur Behandlung eines lebende Zellen enthaltenden biologischen Materials mit einem durch eine Gasentladung bei Atmosphärendruck erzengtem Plasma sind aus der US 5,866,082 A bekannt. Hier ist ein mit Neon gefüllter Glaskolben vorgesehen, der ein Dielektrikum ausbildet. Auf einer Rückseite des Glaskolbens ist eine Elektrode angeordnet, die von einem Wechselhochspannungsgenerator mit einer Wechselhochspannung beaufschlagt wird, um eine Gasentladung einerseits in dem Neongas innerhalb des Glaskolbens und andererseits zwischen dem Glaskolben und einer Hautoberfläche hervorzurufen. Das bekannte Verfahren und die bekannte Vorrichtung dienen zur Behandlung der Haut, über deren Oberfläche die Gasentladung gezündet wird, mit Ozon, welches bei der Gasentladung in Luft entsteht. Die elektrische Leistung, die von dem Wechselhochspannungsgenerator an die Elektrode abgegeben wird, liegt im Bereich mehrerer 10 Watt. Es ist daher wichtig, einen unmittelbaren Kontakt der Haut mit der Elektrode zu vermeiden, auch wenn der dazwischenliegende Gaskolben zerstört wird. Aus diesem Grund steht eine Abschirmung aus elektrisch isolierendem Material nach vorn über die Elektrode in dem Bereich des Glaskolbens über. Die Versorgung der bekannten Vorrichtung mit elektrischer Energie erfolgt über das übliche Stromnetz. Die bekannte Vorrichtung und das bekannte Verfahren sind für die großflächige Behandlung von Haut vorgesehen. Die Abmessungen der Elektrode und des Glaskolbens erlauben keine lokalen Behandlungen. Die Ozonbehandlung der Haut dient kosmetischen Zwecken, indem Bakterien an der Hautoberfläche abgetötet werden.

Eine Weiterentwicklung der aus der US 5,866,082 A bekannten Vorrichtung ist in der GB 2,378,387 A beschrieben. Mit der Weiterentwicklung soll insbesondere dem Problem begegnet werden, das mit einem möglichen Bruch des Glaskolbens verbunden ist. Hierzu ist ein Schutzmantel für den Glaskolben vorgesehen, der Durchbrechungen aufweist, um die Gasentladung gegenüber der Hautoberfläche zu ermöglichen. Dabei ist der GB 2,378,287 zu entnehmen, dass der Glaskolben nicht länger in der Lage ist, elektrische Energie für die Gasentladung zu leiten, wenn er zerbricht und das Neongas entweicht.

Aus der DE 198 20 240 A1 ist ein elektrochirurgisches Instrument bekannt, das ein Plasma durch eine unbehinderte Gasentladung in einem inerten Gas oder Gasgemisch, das keinen Sauerstoff enthält, ausbildet. Die Wirkung des Plasmas auf das mit diesem Instrument behandelten Gewebe ist im Wesentlichen thermisch, d.h. es kommt zur thermischen Koagulation. Eine nicht behinderte elektrische Entladung ist nur im sehr hohen Frequenzbereich und mit relativ hohen elektrischen Leistungen realisierbar.

Im Bereich der Zahnheilkunde ist die Behandlung von Zahnkaries mit Ozon bekannt. Hierzu wird ein abgegrenzter Bereich oberhalb der Stelle eines Zahns, die von Karies befallen ist, mit Ozon durchspült. Das Ozon wird in einer stationären Vorrichtung erzeugt und über einen Schlauch einer Behandlungsglocke an einer Mundsonde zugeführt. Aus derselben Behandlungsglocke wird das Ozon wieder abgesaugt und nicht verbrauchtes Ozon wird in einem Filter neutralisiert. Die bekannte Vorrichtung zur Kariesbehandlung in ihrer Gesamtheit ist recht aufwendig.

Aus der WO 87/07248 ist ein Verfahren zur Behandlung eines lebende Zellen enthaltenden biologischen Materials mit einem durch eine Gasentladung bei Atmosphärendruck erzeugten Plasma bekannt. Hier wird eine Elektrode mit Abstand zu dem biologischen Material angeordnet, das mit einer Gegenelektrode kontaktiert ist. Zum Zünden der Gasentladung zwischen der Elektrode und dem biologischen Material wird eine gleichgerichtete Wechselspannung, die also nur aus Spannungspulsen einer Polarität besteht, zwischen den Elektroden angelegt. Zur flächigeren Behandlung des biologischen Materials, bei dem es sich um menschliche Haut handeln kann, kann die Elektrode oder eine Vielzahl von Elektroden in einen mit Durchbrechungen versehenen dielektrischen Werkstoff eingebettet sein. Eine dielektrische Behinderung der Gasentladung wird hierdurch nicht bewirkt.

Aus der als EP 1 534 099 A1 (WO 2004/023927 A1) nachveröffentlichten europäischen Patentanmeldung sind ein Verfahren und eine Vorrichtung zur Vorbereitung eines Finger- oder Fußnagels für eine Beschichtung, insbesondere Lackierung bekannt. Die Vorbereitung des Finger- oder Fußnagels für die Beschichtung erfolgt, in dem eine Gasentladung unter Atmosphärendruck durch Anlegen einer Wechselhochspannung zwischen dem Nagel und der Elektrode ausgelöst wird, die durch ein vor der Elektrode angeordnetes massives Dielektrikum dielektrisch behindert ist.

### AUFGABE DER ERFINDUNG

Der Erfindung liegt die Aufgabe zugrunde, ein Verfahren und eine Vorrichtung nach den Oberbegriffen der Patentansprüche 1 und 11 aufzuzeigen, mit denen lebende Zellen enthaltende biologische Materialien mit geringem Energieaufwand, mit geringer Unfallgefahr und auch in lokal eng begrenzten Bereichen behandelt werden können.

### LÖSUNG

Die Aufgabe der Erfindung wird durch ein Verfahren mit den Merkmalen des unabhängigen Patentanspruchs 1 und durch eine Vorrichtung mit den Merkmalen des unabhängigen Patentanspruchs 11 gelöst. Vorteilhafte Ausführungsformen des Verfahrens und der Vorrichtung sind in den abhängigen Patentansprüchen 2 bis 10 bzw. 12 bis 20 beschrieben.

### BESCHREIBUNG DER ERFINDUNG

Bei dem neuen Verfahren und der neuen Vorrichtung ist das Dielektrikum ein massives Festkörperdielektrikum, das ohne Abstand vor der Elektrode angeordnet ist, an die die Wechselhochspannung zum Zünden der Gasentladung über dem zu behandelnden biologischen Material angelegt wird. D.h., es gibt keinen aufwändigen gasgefüllten Glaszylinder. Vielmehr ist das Dielektrikum ein einfacher Festkörper. Dabei kann das Dielektrikum durchaus aus Glas bestehen. Es handelt sich dann aber um massives Glas. Das Dielektrikum kann auch aus Keramik oder solchen Kunststoffen bestehen, die gegenüber den Einflüssen der Gasentladung hinreichend inert sind. Verglichen mit einem gasgefüllten Glaskolben kann das Dielektrikum bei dem neuen Verfahren und der neuen Vorrichtung sehr dünn sein. Seine typische Dicke liegt bei wenigen, d.h. maximal 5 mm. Vorzugsweise ist das Dielektrikum maximal 3 mm dick. Je nach Material des Dielektrikums kann aber auch eine Dicke von beispielsweise 0,5 mm und weniger ausreichend sein. Die geringe Dicke des Dielektrikums erleichtert die Ausbildung der Gasentladung über einer kleinen definierten Fläche des zu behandelnden biologischen Materials. Der Verzicht auf jeden zusätzlichen Gasraum zwischen dem Dielektrikum und der Elektrode oder auch innerhalb des Dielektrikums reduziert den Bedarf an elektrischer Energie für die Gasentladung. Die bei der Erfindung dielektrisch behinderte Gasentladung erzeugt ein im Wesentlichen kaltes Plasma, d.h., die thermischen Effekte sind nur gering. Entscheidender sind die chemischen und mikrophysikalischen Effekte, die von dem Plasma verursacht werden. Wenn die Gasentladung in der Anwesenheit von Sauerstoff erfolgt, beruht ein wesentlicher Effekt auf der Erzeugung von freiem Sauerstoff, d.h. atomarem und/oder angeregtem Sauerstoff, der hoch oxidativ ist, so dass beispielsweise Mikroorganismen an der Oberfläche des zu behandelnden biologischen Materials gezielt abgetötet werden können. Bei den abzutötenden Mikroorganismen kann es sich beispielsweise um den Kariesbefall eines Zahns handeln. Freier Sauerstoff ist in dieser Beziehung noch wirksamer als Ozon. Durch die Reaktivität von freiem Sauerstoff können auch Oberflächenschichten von Gewebe, bei dem es sich um entartetes Gewebe handeln kann, abgetötet werden. Trotz der im Wesentlichen nicht thermischen Wirkung des Plasmas bei der Erfindung ist auch eine oxidative Koagulation beispielsweise zur Stillung von Blutungen möglich.

Konkret kann die Gasentladung bei der Erfindung über einer Fläche des biologischen Materials ausgebildet werden, die kleiner als 100 mm², vorzugsweise kleiner als 50 mm² ist. Die von der Gasentladung überspannte Fläche, womit der gesamte Bereich gemeint ist, in dem Gasentladung stattfindet und nicht nur die einzelnen Entladungskanäle, kann auch nur wenige Quadratmillimeter groß sein, so dass eine sehr zielgerichtete lokale Behandlung des biologischen Materials möglich ist.

Um jegliche thermischen Einflüsse des Plasmas von dem zu behandelnden biologischen Material fernzuhalten, kann eine Gasströmung im Bereich der Gasentladung über das biologische Material geführt werden, die das biologische Material kühlt. Mit der Gasströmung können auch Reaktionsprodukte aus dem Bereich des Plasmas abgeführt werden.

Um freien Sauerstoff nicht unkontrolliert in die Umgebung der Gasentladung gelangen zu lassen, ist es bevorzugt, Gas aus dem Bereich der Gasentladung abzusaugen. Dabei kann die Absaugung koaxial zu der Elektrode erfolgen, also beispielsweise durch einen Ringraum um die Elektrode oder durch ein von der Elektrode gebildetes Rohr, so dass die Vorrichtung im Bereich der Gasentladung trotz der Gasentladung sehr schmal baut.

Die Gasströmung kann aber auch dazu genutzt werden, die Gasentladung in einem Gas oder Gasgemisch zu zünden, dessen Zusammensetzung von Luft abweicht, um bestimmte Reaktionen des zu behandelnden biologischen Materials mit bestimmten Inhaltsstoffen des Gases oder Gasgemisches gezielt hervorzurufen.

Die elektrische Leistung der Gasentladung beträgt bei der Erfindung weniger als 10 Watt Vorzugsweise ist die elektrische Leistung noch geringer und liegt unterhalb von 5 Watt. Sie kann konkret bei einem Watt oder noch darunter liegen.

Besonders bevorzugt ist es, wenn die Wechselhochspannung mit einer Frequenz im Bereich von 1 bis 3.000 kHz in Form einzelner bipolarer Spannungspulse der Größenordnung 1 kV erzeugt wird. Dies ist mit handelsüblichen elektronischen Halbleiterbausteinen möglich. D.h., ein Wechselhochspannungsgenerator der neuen Vorrichtung kann vergleichsweise preisgünstig realisiert werden. Durch die Bipolarität der Pulse ist es nicht zwingend erforderlich, das biologische Material und den Wechselhochspannungsgenerator zu erden oder einen Rückflussstromkreisiauf zwischen ihnen bereitzustellen. Diese Maßnahmen können aber dennoch im Rahmen der Erfindung ergriffen werden.

Um einen unerwünschten Kontakt von biologischem Material mit der aktiven Fläche des Dielektrikums zu verhindern, kann der Bereich der Gasentladung seitlich mit elektrisch isolierendem und sich nicht statisch aufladendem Material abgeschirmt werden. Dies ist beispielsweise dann sinnvoll, wenn die Gasentladung in einem Mundraum zur Behandlung eines mit Karies befallenen Zahns gezündet wird und dabei die Gefahr besteht, dass die Zunge des Patienten mit dem Dielektrikum in Kontakt gerät. Auch ein unmittelbarer Kontakt des zu behandelnden Zahns mit dem Dielektrikum ist unerwünscht.

Als besonderer Vorteil der vorliegenden Erfindung ergibt sich, dass diese im Batteriebetrieb umgesetzt werden kann. D.h., die Wechselspannung zum Zünden und Aufrechterhalten der Gasentladung kann unter Verwendung elektrischer Energie aus einem handelsüblichen Akkumulator erzeugt werden. Damit ist die neue Vorrichtung zur Durchführung des neuen Verfahrens als kompaktes Handgerät ausbildbar.

Sobald im Zusammenhang mit der Definition der neuen Vorrichtung in den Patentansprüchen 11 bis 20 auf eine aktive Fläche des Dielektrikums verwiesen wird, so handelt es sich um die dem biologischen Material bei der Verwendung der Vorrichtung zugewandte Oberfläche des Dielektrikums, über der sich beim Anlegen der Wechselhochspannung an die Elektrode eine elektrische Feldstärke ausbildet, die zum Zünden und Aufrechterhalten der Gasentladung ausreichend ist. Was im Einzelfall als aktive Oberfläche des Dielektrikums anzusehen ist, hängt von der Geometrie der Elektrode und des

Dielektrikums ab und wird natürlich auch von der an die Elektrode angelegten Wechselhochspannungen beeinflusst. Praktisch ist der Umfang der aktiven Fläche des Dielektrikums aber leicht festzustellen, indem geschaut wird, wo vor der Oberfläche des Dielektrikums die Gasentladung erfolgt.

Konkrete Anwendungen des neuen Verfahrens und der neuen Vorrichtung schließen die Behandlung von Juckreiz an der Haut, wie er beispielsweise bei Neurodermitis und auch bei Mückenstichen auftritt, ein. Nach der Plasmabehandlung geht ein solcher Juckreiz deutlich zurück. Das Abtöten von Viren bei der Hautbehandlung kann beispielsweise dazu genutzt werden, um Warzen, Gürtelrose oder Herpes zu behandeln. Bei der Zahnbehandlung mit dem neuen Verfahren und der neuen Vorrichtung kann ein Zahn auch für die Kariesprophylaxe vorbereitet werden, indem er mit dem Plasma von Speichelresten gereinigt wird, Bakterien und Viren abgetötet werden und seine Zahnoberfläche aktiviert bzw. die Oberflächenenergie des Zahns erhöht wird. Diese Vorbehandlung bewirkt, dass eine nachfolgende Lackierung des Zahns mit einem Fluorprotektor, einer Fisurenversiegelung oder dergleichen besser wirkt und länger hält. Die Beschichtung des Zahns kann auch durch das Plasma selbst bewirkt werden, wenn dem Gas, in dem die Gasentladung zur Erzeugung des Plasmas gezündet wird, beispielsweise Methan und Silan zugesetzt werden. Es können auch fluorierte Gase wie Tetrafluormethan zugeführt werden.

### KURZBESCHREIBUNG DER FIGUREN

Im Folgenden wird die Erfindung anhand von in den Figuren dargestellten bevorzugten Ausführungsbeispielen weiter erläutert und beschrieben.
- **Fig. 1**: zeigt schematisch den Aufbau einer ersten Ausführungsform der neuen Vorrichtung bei der Durchführung des neuen Verfahrens,
- **Fig. 2**: zeigt schematisch den Aufbau einer zweiten Ausführungsform der neuen Vorrichtung bei der Durchführung einer zweiten Ausführungsform des neuen Verfahrens.
- **Fig. 3**: zeigt schematisch den Aufbau einer weiteren Ausführungsform der neuen Vorrichtung bei der Durchführung einer weiteren Ausführungsform des neuen Verfahrens und
- **Fig. 4**: zeigt schematisch den Aufbau noch einer weiteren Ausführungsform der neuen Vorrichtung bei der Durchführung noch einer weiteren Ausführungsform des neuen Verfahrens.

### FIGURENBESCHREIBUNG

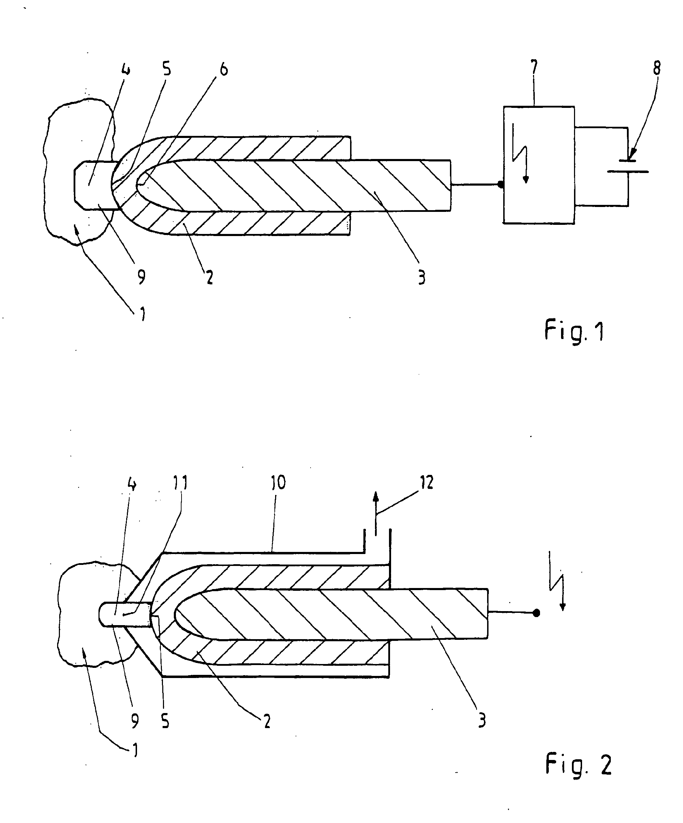

Die in Fig. 1 dargestellte Vorrichtung weist als wesentlichen Bestandteil eine stiftförmige Elektrode 3, ein die abgerundete Spitze 6 der Elektrode 3 abdeckendes Dielektrikum 2 und einen Wechselhochspannungsgenerator 7 auf, der eine Wechselhochspannung erzeugt, welche im Betrieb der Vorrichtung an der Elektrode 3 anliegt. Diese Bestandteile der Vorrichtung können gemeinsam mit einem oder mehreren Akkumulatoren 8 zur Versorgung des Wechselhochspannungsgenerators 7 mit elektrischer Energie in einem Handgerät untergebracht sein. Der Wechselhochspannungsgenerator 7 kann aber auch über ein Netzgerät mit elektrischer Energie versorgt werden. Dabei kann das Netzgerät oder eine Einheit aus dem Netzgerät und dem Wechselhochspannungsgenerator als Standgerät ausgebildet sein. Das Dielektrikum 2 dient einerseits zur Isolierung der Elektrode 3. Andererseits dient es zur dielektrischen Behinderung einer Gasentladung 9, welche durch Anlegen der Wechselhochspannung an die Elektrode 3 zwischen dem Dielektrikum 2 und der Oberfläche eines biologischen Materials 1 gezündet werden kann, und die ein Plasma 4 über der Oberfläche des biologischen Materials 1 erzeugt. Wenn die Gasentladung in der Anwesenheit von Sauerstoff, beispielsweise Luftsauerstoff erfolgt, umfasst das Plasma freien Sauerstoff, d.h. hochreaktive freie Sauerstoffatome, die chemisch auf das biologische Material 1 an seiner Oberfläche einwirken. Die dielektrische Behinderung der Gasentladung 9 durch das Dielektrikum 2 resultiert in ein kaltes Plasma 4. D.h., die thermischen Effekte des Plasmas 4 sind zumindest über kürzere Einwirkzeiten auf das biologische Material 1 von wenigen bis einigen Sekunden vernachlässigbar. Die Querschnittsfläche der Gasentladung 9 wird durch den Bereich definiert, in dem eine ausreichende elektrische Feldstärke zum Aufrechterhalten der Gasentladung 9 zwischen dem Dielektrikum 2 und dem biologischen Material 1 vorliegt. Die entsprechende Oberfläche des Dielektrikums 2 wird hier auch als aktive Fläche des Dielektrikums 2 bezeichnet. Außerhalb dieser aktiven Fläche dient das Dielektrikum 2 vorwiegend als Isolierung der Elektrode 3. Es kann dazu außerhalb der aktiven Fläche auch gezielt eine andere Zusammensetzung und/oder höhere Wandstärke aufweisen. Im Bereich der aktiven Fläche des Dielektrikums 2 beträgt seine Wandstärke typischerweise einige wenige Millimeter. Das Material des Dielektrikums 2 ist vorzugsweise eine Keramik. Es kann sich aber auch um Glas oder einen gegenüber dem Plasma 4 hinreichend resistenten Kunststoff handeln. Die Behandlung des biologischen Materials 1 durch das Plasma 4 besteht im Wesentlichen darin, Zellen, beispielsweise unerwünschte Mikroorganismen wie Bakterien oder entartetes Gewebe, an der Oberfläche des biologischen Materials 1 abzutöten. Konkret kann dies zur Behandlung von Karies an einem Zahn erfolgen.

Die in Fig. 2 dargestellte Ausführungsform der neuen Vorrichtung unterscheidet sich von derjenigen gemäß Fig. 1 zunächst dadurch, dass die Details bezüglich des Wechselhochspannungsgenerators und seiner Versorgung mit elektrischer Energie weggelassen sind. Entscheidend ist jedoch, dass hier ein Absaug- und Isoliergehäuse mit Abstand um das Dielektrikum 2 herum angeordnet ist, das vor der aktiven Fläche des Dielektrikums 2 eine Öffnung 11 aufweist, durch die hindurch Gas aus dem Bereich der Gasentladung 9 abgesaugt wird. Durch diese Absaugung wird das unkontrollierte Freisetzen von freiem Sauerstoff in die Umgebung der Gasentladung 9 aus dem Plasma 4 heraus verhindert. Vielmehr kann solcher Sauerstoff nach der Absaugung in geeigneten Filtern neutralisiert werden. Das Material des Isolier- und Absauggehäuses 10 ist derart elektrisch isolierend und nicht statisch aufladbar, dass sichergestellt ist, dass weder eine Gasentladung in dem Zwischenraum zwischen dem Dielektrikum 2 und dem Isolier- und Absauggehäuse 10 gezündet wird, noch dass ein direkter Kontakt mit dem Isolier- und Absauggehäuse 10 selbst mit einem leitenden Gegenstand zur Übertragung von elektrischer Energie führt. So kann beispielsweise bei der Behandlung von Karies im Mundraum verhindert werden, dass die Zunge des Patienten mit dem Dielektrikum 2 oder gar der Elektrode 3 in Kontakt kommt, was zu einer unangenehmen elektrischen Reizung führen würde.

Statt der Absaugung von Gas 12 aus dem Bereich der Gasentladung 9 kann in den diesen Bereich auch gezielt ein Gas zugeführt werden, dessen Zusammensetzung von Luft abweicht oder dass auch allein zur Kühlung der Oberfläche des biologischen Materials 1 dient. Eine solche Kühlung wird aber auch durch die Absaugung von Gas 12 bei der Vorrichtung gemäß Fig. 2 erreicht. In jedem Fall bewirkt die Kühlung der Oberfläche des biologischen Materials 1 durch eine Gasströmung, dass sich die Auswirkungen des Plasmas 4 auf nichtthermische chemische und mikrophysikalische Effekte beschränken.

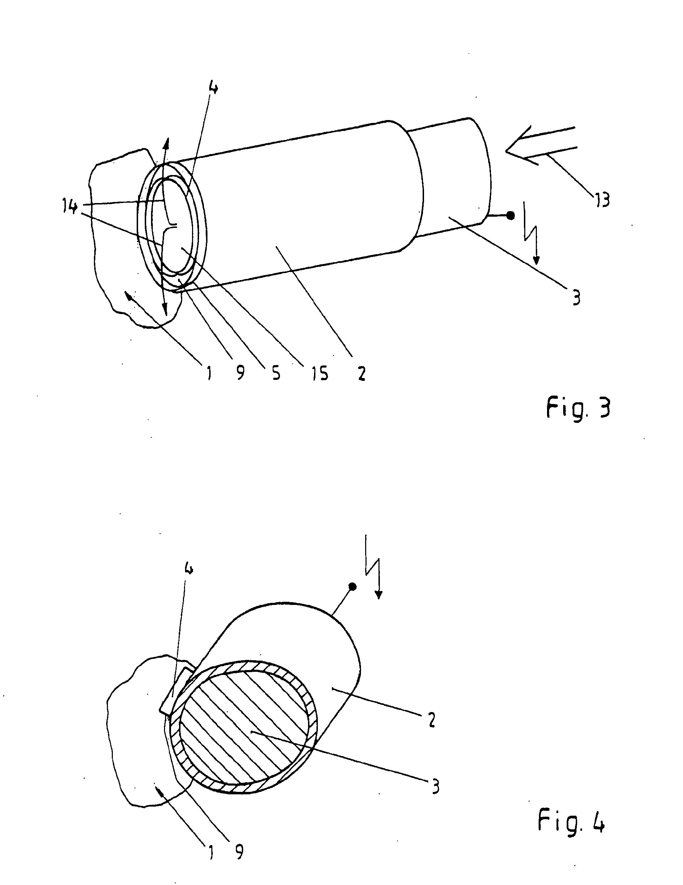

Bei der Ausführungsform der Vorrichtung gemäß Fig. 3 wird ein Reaktionsgas 13 mit einer speziellen von Luft abweichenden Zusammensetzung in den Bereich der Gasentladung 9 eingeblasen. Dies erfolgt wie in Fig. 2 in einer koaxialen Anordnung. In Fig. 3 ist aber die Elektrode 3 rohrförmig und sowohl innen als auch außen sowie an ihrer Spitze 6 mit dem Dielektrikum 2 überzogen. Die resultierende aktive Fläche des Dielektrikums 2 ist eine Ringfläche. D.h., das Gebiet der Gasentladung 9 ist zylindermantelförmig. Zusätzlich zu der Zufuhr von Reaktionsgas 13 könnte in einer Fig. 2 entsprechenden Anordnung auch noch Gas aus dem Bereich der Gasentladung 9 abgesaugt werden, um auch hier das unkontrollierte Freisetzen beispielsweise von freiem Sauerstoff in die Umgebung der Gasentladung 9 zu unterbinden.

Fig. 4 zeigt eine Ausführungsform der Vorrichtung mit etwas anderer geometrischer Anordnung. Hier ist die stabförmige Elektrode 3 umlaufend von dem Dielektrikum 2 umgeben. Bei paralleler Anordnung der Elektrode 3 zu der Oberfläche des biologischen Materials 1 bildet sich so ein linienförmiger Bereich der Gasentladung 9 aus, in dem das Plasma 4 erzeugt wird. Durch Bewegen der Elektrode 3 über die Oberfläche des biologischen Materials 1 können so relativ große Flächen des biologischen Materials 1 mit dem Plasma 4 behandelt werden, obwohl die zu einem Zeitpunkt behandelte Oberfläche des biologischen Materials 1, die der aktiven Fläche des Dielektrikums 2 entspricht, immer nur vergleichsweise klein ist. Die geringe Größe des Gebiets der Gasentladung 9 reduziert ebenso wie die dielektrische Behinderung der Gasentladung 9 den Energiebedarf für die Gasentladung 9, was Voraussetzung dafür ist, dass die Gasentladung 9 unter Verwendung von Akkumulatoren, konkret handelsüblichen Batterien, als elektrische Energieversorgung möglich ist. Gleichzeitig stellt die geringe elektrische Leistung der Gasentladung 9 auch sicher, dass das Niveau jeglicher elektrischer Reizungen, die mit der Verwendung der neuen Vorrichtung, d.h. der Anwendung des neuen Verfahrens auch im Extremfall verbunden sind, klein bleibt. Dies ist ein wichtiger Sicherheitsaspekt. Ein zusätzlicher Sicherheitsaspekt besteht darin, dass das Dielektrikum 2 ein massives Festkörperdielektrikum ist, das nahtlos auf der Elektrode 3 angeordnet ist, so dass die Gefahr eines direkten Kontakts mit der Elektrode 3 auch bei einer unsachgemäßen Verwendung der neuen Vorrichtung realistisch nicht vorhanden ist.

### BEZUGSZEICHENLISTE

- 1: biologisches Material
- 2: Dielektrikum
- 3: Elektrode
- 4: Plasma
- 5: aktive Fläche
- 6: Spitze
- 7: Wechselhochspannungsgenerator
- 8: Akkumulator
- 9: Gasentladung
- 10: Isolier- und Absauggehäuse
- 11: Öffnung
- 12: Gas
- 13: Reaktionsgas
- 14: Gasströmung
- 15: Gaszuführung

## Patentansprüche

1. Verfahren zur Behandlung eines lebende Zellen enthaltenden biologischen Materials mit einem durch eine Gasentladung bei Atmosphärendruck erzeugten Plasma, wobei eine Elektrode mit Abstand zu dem biologischen Material angeordnet wird, wobei ein Dielektrikum mit Abstand zu dem biologischen Material zwischen der Elektrode und dem biologischen Material angeordnet wird und wobei zum Zünden der durch das Dielektrikum dielektrisch behinderten Gasentladung zwischen dem Dielektrikum und dem biologischen Material eine bipolare Wechselhochspannung an die Elektrode angelegt wird, **dadurch gekennzeichnet, dass** als Dielektrikum (2) ein massives Festkörperdielektrikum ohne Abstand vor der Elektrode (3) angeordnet wird und dass die Vechselhochspannung so an die Elektrode (3) angelegt wird, dass die elektrische Leistung der Gasentladung weniger als 10W beträgt.

2. Verfahren nach Anspruch 1, **dadurch gekennzeichnet, dass** die Gasentladung (9) über einer Fläche des biologischen Materials (1) ausgebildet wird, die kleiner als 100 mm², vorzugsweise kleiner als 50 mm², ist.

3. Verfahren nach Anspruch 1 oder 2, **dadurch gekennzeichnet, dass** eine Gasströmung (14) im Bereich der Gasentladung (9) über das biologische Material (1) geführt wird.

4. Verfahren nach einem der Ansprüche 1 bis 3, **dadurch gekennzeichnet, dass** Gas (12) aus dem Bereich der Gasentladung (9) abgesaugt wird.

5. Verfahren nach Anspruch 4, **dadurch gekennzeichnet, dass** das Gas (12) koaxial zu der Elektrode (2) abgesaugt wird.

6. Verfahren nach einem der Ansprüche 1 bis 5, **dadurch gekennzeichnet, dass** die Gasentladung (9) in einem Gas oder Gasgemisch (13) gezündet wird, dessen Zusammensetzung von Luft abweicht.

7. Verfahren nach einem der Ansprüche 1 bis 6, **dadurch gekennzeichnet, dass** die Wechselhochspannung so an die Elektrode (3) angelegt wird, dass die elektrische Leistung der Gasentladung (9) weniger als 5 W beträgt.

8. Verfahren nach einem der Ansprüche 1 bis 7, **dadurch gekennzeichnet, dass** die Wechselhochspannung mit einer Frequenz von 1 bis 3000 kHz in Form einzelner bipolarer Spannungspulse der Größenordnung 1 kV erzeugt wird.

9. Verfahren nach einem der Ansprüche 1 bis 8, **dadurch gekennzeichnet, dass** der Bereich der Gasentladung (9) seitlich mit elektrisch isolierendem und sich nicht statisch aufladenden Material (10) abgeschirmt wird.

10. Verfahren nach einem der Ansprüche 1 bis 9, **dadurch gekennzeichnet, dass** die Wechselhochspannung unter Verwendung elektrischer Energie aus einem Akkumulator (8) erzeugt wird.

11. Vorrichtung zur Behandlung eines lebende Zellen enthaltenden biologischen Materials, insbesondere nach dem Verfahren nach einem der Ansprüche 1 bis 10, mit einem durch eine Gasentladung bei Atmosphärendruck erzeugten Plasma, mit einer Elektrode, mit einem vor der Elektrode angeordneten Dielektrikum und mit einem Wechselhochspannungsgenerator zum Erzeugen einer an der Elektrode anliegenden bipolaren Wechselhochspannung, die die durch das Dielektrikum dielektrisch behinderte Gasentladung zwischen einer aktiven Fläche des Dielektrikums und dem biologischen Material zündet, **dadurch gekennzeichnet, dass** das Dielektrikum (2) ein massives Festkörperdielektrikum ist, das ohne Abstand vor der Elektrode (3) angeordnet ist und dass der Wechselhochspannungsgenerator (7) eine elektrische Leistung von weniger als 10W an die Elektrode (3) abgibt.

12. Vorrichtung nach Anspruch 11, **dadurch gekennzeichnet, dass** die aktive Fläche (5) des Dielektrikums (2) kleiner als 100 mm², vorzugsweise kleiner als 50 mm², ist.

13. Vorrichtung nach Anspruch 11 oder 12, **dadurch gekennzeichnet, dass** eine Gaszuführung (15) in den Bereich vor der aktiven Fläche (5) des Dielektrikums (2) vorgesehen ist

14. Vorrichtung nach einem der Ansprüche 1 bis 5, **dadurch gekennzeichnet. dass** die Gaszuführung (15) an ein Reservoir für ein Gas oder Gasgemisch (13) mit anderer Zusammensetzung als Luft angeschlossen ist.

15. Vorrichtung nach einem der Ansprüche 11 bis 14, **dadurch gekennzeichnet, dass** eine Gasabsaugung (10) aus dem Bereich vor der aktiven Fläche (5) des Dielektrikums (2) vorgesehen ist.

16. Vorrichtung nach Anspruch 15, **dadurch gekennzeichnet, dass** Gasabsaugung (10) ein koaxial zu der Elektrode (3) angeordnetes Absaugrohr aufweist.

17. Vorrichtung nach einem der Ansprüche 11 bis 16, **dadurch gekennzeichnet, dass** der Wechselhochspannungsgenerator (7) eine elektrische Leistung von weniger als 5 W an die Elektrode (3) abgibt.

18. Vorrichtung nach einem der Ansprüche 11 bis 17, **dadurch gekennzeichnet, dass** der Wechselhochspannungsgenerator (7) die Wechselhochspannung mit einer Frequenz von 1 bis 3000 kHz in Form einzelner bipolarer Spannungspulse der Größenordnung 1 kV erzeugt.

19. Vorrichtung nach einem der Ansprüche 11 bis 18, **dadurch gekennzeichnet, dass** eine seitliche Abschirmung (10) aus elektrisch isolierendem und sich nicht statisch aufladenden Material über die aktive Fläche (5) des Dielektrikums (2) vorsteht.

20. Vorrichtung nach einem der Ansprüche 1 bis 9, **dadurch gekennzeichnet, dass** der Wechselhochspannungsgenerator (7) und die Elektrode (3) mit dem Dielektrikum (2) Teile eines batteriebetriebenen Handgeräts sind.

## Claims

1. A method of treating a biological material containing living cells with a plasma generated by a gas discharge at atmospheric pressure, wherein an electrode is arranged at a distance to the biological material, wherein a dielectric is arranged between the electrode and the biological material at a distance to the biological material, and wherein a bipolar alternating high voltage is applied to the electrode for igniting a gas discharge dielectrically barred by the dielectric between the dielectric and the biological material, **characterized in that** a compact solid body dielectric (2) is arranged at zero distance in front of the electrode (3), and that the alternating high voltage is applied to the electrode (3) in such a way that the electric power of the gas discharge is lower than 10 W.

2. The method of claim 1, **characterized in that** the gas discharge (9) is formed over an area of the biological material (1) which is smaller than 100 mm², preferably smaller than 50 mm².

3. The method of claim 1 or 2, **characterized in that** a gas flow (14) is guided over the biological material (1) in the region of the gas discharge (9).

4. The method of any of the claims 1 to 3, **characterized in that** gas (12) is sucked out of the region of the gas discharge (9).

5. The method of claim 4, **characterized in that** the gas (12) is sucked off coaxially with regard to the electrode (2).

6. The method of any of the claims 1 to 5, **characterized in that** the gas discharge is ignited in a gas or gas mixture, the composition of which deviates from air.

7. The method of any of the claims 1 to 6, **characterized in that** the alternating high voltage is applied to the electrode (3) in such a way that the electric power of the gas discharge (9) is smaller than 5 W.

8. The method of any of the claims 1 to 7, **characterized in that** the alternating high voltage is generated at a frequency of 1 to 3,000 kHz as individual bipolar voltage pulses of an order of magnitude of 1 kV.

9. The method of any of the claims 1 to 8, **characterized in that** the region of the gas discharge (9) is laterally shielded with a material (10) which is electrically isolating and not subject to electrostatic charging.

10. The method of any of the claims 1 to 9, **characterized in that** the alternating high voltage is generated using electric energy out of an accumulator (8).

11. An apparatus for treating a biological material containing living cells, partivularly according to the method of any of the claims 1 to 10, with a plasma generated by a gas discharge at atmospheric pressure, the apparatus comprising an electrode, a dielectric arranged in front of the electrode, and a alternating high voltage generator for generating a bipolar alternating high voltage connected to the electrode, which ignites a gas discharge dielectrically barred by the dielectric between an active area of the dielectric and the biological material, **characterized in that** the dielectric (2) is a compact solid body dielectric which is arranged at zero distance in front of the electrode (3), and that the alternating high voltage generator (7) outputs an electric power of less than 10 W to the electrode (3).

12. The apparatus of claim 11, **characterized in that** the active area (5) of the dielectric (2) is smaller than 100 mm², preferably smaller than 50 mm².

13. The apparatus of claim 1 or 12, **characterized in that** a gas supply (15) is provided into the region in front of the active area (5) of the dielectric (2).

14. The apparatus of claim 13, **characterized in that** the gas supply (15) is connected to a reservoir of a gas or gas mixture (13) having another composition than air.

15. The apparatus of any of the claims 11 to 14, **characterized in that** a gas exhaustion (10) is provided out of the region in front of the active area (5) of the dielectric (2).

16. The apparatus of claim 15, **characterized in that** the gas exhaustion (10) comprises an exhaust tube which is arranged coaxially with regard to the electrode (3).

17. The apparatus of any of the claims 11 to 16, **characterized in that** the alternating high voltage generator (7) outputs an electric power of less than 5 W to the electrode (3).

18. The apparatus of any of the claims 11 to 17, **characterized in that** the alternating high voltage generator (7) generates the alternating high voltage at a frequency of 1 to 3,000 kHz as individual bipolar voltage pulses of an order of magnitude of 1 kV.

19. The apparatus of any of the claims 11 to 18, **characterized in that** a lateral shielding (10) made of a material which is electrically isolating and not subject to electrostatic charging laterally protrudes beyond the active area (5) of the dielectric (2).

20. The apparatus of any of the claims 11 to 19, **characterized in that** the alternating high voltage generator (7) and the electrode (3) with the dielectric (2) are parts of a battery powered handheld unit.

## Revendications

1. Procédé de traitement d'un matériau biologique contenant des cellules vivantes avec un plasma produit par décharge gazeuse à la pression atmosphérique, une électrode étant disposée à distance du matériau biologique, un diélectrique étant disposé à distance du matériau biologique entre l'électrode et le matériau biologique et une haute tension alternative étant appliquée sur l'électrode pour amorcer la décharge gazeuse empêchée de manière diélectrique par le diélectrique entre le diélectrique et le matériau biologique, **caractérisé en ce qu'**un diélectrique solide et massif comme diélectrique (2) est disposé à proximité de l'électrode (3) et **en ce que** la haute tension alternative est appliquée sur l'électrode (3) de sorte que la puissance électrique de la décharge gazeuse s'élève à moins de 10 W.

2. Procédé selon la revendication 1, **caractérisé en ce que** la décharge gazeuse (9) est réalisée sur une surface du matériau biologique (1) qui est inférieure à 100 mm², de préférence inférieure à 50 mm².

3. Procédé selon la revendication 1 ou 2, **caractérisé en ce qu'**un courant de gaz (14) est guidé dans la zone de la décharge gazeuse (9) au-dessus du matériau biologique (1).

4. Procédé selon l'une quelconque des revendications 1 à 3, **caractérisé en ce que** le gaz (12) est évacué de la zone de la décharge gazeuse (9).

5. Procédé selon la revendication 4, **caractérisé en ce que** le gaz (12) est évacué de manière coaxiale par rapport à l'électrode (2).

6. Procédé selon l'une quelconque des revendications 1 à 5, **caractérisé en ce que** la décharge gazeuse (9) est amorcée dans un gaz ou mélange de gaz (13) dont la composition s'écarte de celle de l'air.

7. Procédé selon l'une quelconque des revendications 1 à 6, **caractérisé en ce que** la haute tension alternative est appliquée sur l'électrode (3) de sorte que la puissance électrique de la décharge gazeuse (9) s'élève à moins de 5 W.

8. Procédé selon l'une quelconque des revendications 1 à 7, **caractérisé en ce que** la haute tension alternative est produite à une fréquence allant de 1 à 3000 kHz sous forme d'une impulsion de tension bipolaire unique de l'ordre de 1 kV.

9. Procédé selon l'une quelconque des revendications 1 à 8, **caractérisé en ce que** la zone de la décharge gazeuse (9) est protégée latéralement par un matériau (1) électriquement isolant et sans décharge statique.

10. Procédé selon l'une quelconque des revendications 1 à 9, **caractérisé en ce que** la haute tension alternative est produite en utilisant l'énergie électrique provenant d'un accumulateur (8).

11. Dispositif de traitement d'un matériau biologique contenant des cellules vivantes, en particulier d'après le procédé selon l'une quelconque des revendications 1 à 10, avec un plasma produit par une décharge gazeuse à la pression atmosphérique, comprenant une électrode, comprenant un diélectrique disposé devant l'électrode et comprenant un générateur de haute tension alternative destiné à produire une haute tension alternative bipolaire appliquée sur l'électrode, qui amorce la décharge gazeuse empêchée de manière diélectrique par le diélectrique entre une surface active du diélectrique et le matériau biologique, **caractérisé en ce que** le diélectrique (2) est un diélectrique solide et massif qui est disposé à proximité de l'électrode (3) et **en ce que** le générateur (7) de la haute tension alternative délivre à l'électrode (3) une puissance électrique inférieure à 10 W.

12. Dispositif selon la revendication 11, **caractérisé en ce que** la surface active (5) du diélectrique (2) est inférieure à 100 mm², de préférence inférieure à 50 mm².

13. Dispositif selon la revendication 11 ou 12, **caractérisé en ce qu'**une arrivée de gaz (15) est prévue dans la zone de la surface active (5) du diélectrique (2).

14. Dispositif selon la revendication 13, **caractérisé en ce que** l'arrivée de gaz (15) est raccordée à un réservoir de gaz ou de mélange de gaz (13) présentant une autre composition que celle de l'air.

15. Dispositif selon l'une quelconque des revendications 11 à 14, **caractérisé en ce qu'**une évacuation de gaz (10) est prévue à l'extérieur de la zone devant la surface active (5) du diélectrique (2).

16. Dispositif selon la revendication 15, **caractérisé en ce que** l'évacuation de gaz (10) comprend un tube d'évacuation disposé de manière coaxiale par rapport à l'électrode (3).

17. Dispositif selon l'une quelconque des revendications 11 à 16, **caractérisé en ce que** le générateur de haute tension alternative (7) délivre une puissance électrique inférieure à 5 W à l'électrode (3).

18. Dispositif selon l'une quelconque des revendications 11 à 17, **caractérisé en ce que** le générateur de haute tension alternative (7) produit la haute tension alternative à une fréquence de 1 à 3000 kHz sous forme d'une impulsion de tension bipolaire unique de l'ordre de 1 kV.

19. Dispositif selon l'une quelconque des revendications 11 à 18, **caractérisé en ce qu'**un écran latéral (10) en matériau électriquement isolant et sans décharge statique dépasse de la surface active (5) du diélectrique (2).

20. Dispositif selon l'une quelconque des revendications 11 à 19, **caractérisé en ce que** le générateur de haute tension alternative (7) et l'électrode (3) avec le diélectrique (2) sont les pièces d'un appareil portatif alimenté par une batterie.
